Europäisches Patentamt

European Patent Office

Office européen des brevets

(19)

(11) **EP 1 114 611 A2**

(12) **EUROPÄISCHE PATENTANMELDUNG**

(43) Veröffentlichungstag:
**11.07.2001  Patentblatt 2001/28**

(51) Int Cl.[7]: **A61B 5/08**

(21) Anmeldenummer: **00128603.8**

(22) Anmeldetag: **28.12.2000**

(84) Benannte Vertragsstaaten:
**AT BE CH CY DE DK ES FI FR GB GR IE IT LI LU
MC NL PT SE TR**
Benannte Erstreckungsstaaten:
**AL LT LV MK RO SI**

(30) Priorität: **30.12.1999  DE 19964050**

(71) Anmelder: **Albert, Alfred
97688 Bad Kissingen (DE)**

(72) Erfinder: **Albert, Alfred
97688 Bad Kissingen (DE)**

(74) Vertreter: **Götz, Georg, Dipl.-Ing. et al
Götz, Küchler, Dameron
Patent- und Rechtsanwälte
Postfach 11 93 40
90103 Nürnberg (DE)**

(54) **Simulationsverfahren zum Test von Single-Breath-Diffusion-Messeinrichtungen**

(57)     System zur Simulation eines CO Diffusionsmanövers bestehend aus einem manuell oder elektrisch befüllten Mischbehälter, gegebenenfalls mit Pumpenfunktion, mit steuerbaren Ventilen und einer Volumenmeßeinrichtung, wobei der Mischbehälter volumengesteuert und/oder volumenabhängig mit verschiedenen Testgasen gefüllt wird, und dieses Gasgemisch der zu überprüfenden Meßeinrichtung zugeführt wird, welche dann funktions- und / oder bestimmungsgemäß verwendet wird.

EP 1 114 611 A2

**Beschreibung**

Die Messung der CO Diffusionskapazität hat inzwischen große Bedeutung in der Lungenfunktionsdiagnostik. Die Single-Breath-( Einatemzugmethode)-Technik wird dabei wegen der unkomplizierten Durchführung überwiegend eingesetzt.

Die Genauigkeit der im Markt eingesetzten Geräte kann jedoch kaum oder nur mit großem Aufwand überprüft werden, da diese Geräte nicht portabel sind.

Zur Lösung dieses Problems wird ein System vorgeschlagen, bei dem die Atmung des Probanden durch eine mechanische oder elektrische Pumpe, die als Mischbehälter dient, simuliert wird. Der Gasaustausch wird durch definierte Zugabe verschiedener Testgase simuliert.

Das Atemmanöver ist für die marktüblichen Meßgeräte ähnlich.

Das Atemmanöver mit einem menschlichen Testprobanden läuft folgendermaßen ab:

Der Proband atmet nach einer kurzen Ruheatmungsphase tief aus. Nach der tiefen Ausatmung wird mit Beginn der Einatmung von Raumluft auf ein Testgasgemisch umgeschaltet. Der Proband atmet das Testgas tief ein und hält die Luft für etwa 10 Sekunden an. Anschließend atmet der Proband tief aus. Während der Ausatmung werden ca. 0.51 des ausgeatmeten Gases für eine Analyse gesammelt.

Hintergrund der Methode: Der Proband atmet ein Testgas bestehend aus einem Inert- und Diffusionsgas ein.

Während er 10 Sekunden die Luft anhält, vermischt sich das Inertgas gleichmäßig in der Lunge mit dem vorhandenen Residualvolumen. Das Diffusionsgas verteilt sich gleichfalls, diffundiert jedoch gleichzeitig in das Blut des Probanden. Die Menge des diffundierten Gases hängt von der Zeit ab.

Um die Diffusionskapazität rechnerisch zu bestimmen, wird die eingeatmete Diffusiongaskonzentration mit der ausgeatmeten Diffusionsgaskonzentration verglichen. Ebenso werden die Inertgaskonzentrationen bei Ein- und Ausatmung verglichen und ins Verhältnis mit den Diffusionsgaskonzentrationen gesetzt. Die daraus resultierende Differenzmenge an Diffusionsgas, im Verhältnis mit der luftangehaltenen Zeit ist als Diffusionskapazität definiert.

Diffusionskapazität=

(Konstante / Zeit) * log ( eingeatmete Diffusionsgaskonzentration *

ausgeatmete Inertgaskonzentration /

ausgeatmete Diffusionsgaskonzentration /

eingeatmete Inertgaskonzentration )

Die Konstante =53.6 und ist bekannt
Zeit = die angehaltene Luftanhaltezeit

Als Simulator wird folgende Anordnung vorgeschlagen:

Ein manuell oder elektrisch betriebener Mischbehälter. Der Mischbehälter kann volumengesteuert wahlweise mit verschiedenen Gasen gefüllt werden.

Funktionsprinzip anhand eines Beispiels:

Die angegebenen Füllmengen in % beziehen sich auf das gesamte Mischbehältervolumen.
Der Mischbehälter wird zu 20% mit Raumluft gefüllt (1. Ventil 'siehe unten').

Diese 20% Raumluft setzen sich zusammen aus 0% Inertgas, 0% Diffusionsgas, 21% 02, 79% N2.

Anschließend werden weitere 50% Testgas in den Mischbehälter eingefüllt (2. Ventil 'siehe unten'). Diese 50% Testgas bestehen für sich genommen zu 20% aus Inertgas, zu 0.3% aus Diffusionsgas, zu 21% aus 02, und zu 58.7% aus N2.

Zuletzt werden weitere 30%, bezogen auf das gesamte Mischbehältervolumen eines zweiten Testgases eingefüllt (2. Ventil 'siehe unten').
Diese 30% des zweiten Testgases bestehen für sich genommen zu 20% aus Inertgas, zu 0% aus Diffusionsgas, zu 21% aus 02, zu 59% aus N2.

Das zuletzt eingefüllte Testgas täuscht das Verschwinden des Diffusionsgases vor (siehe obiges Verfahren beim Probanden). Nach 10 Sekunden wird der Mischbehälter wieder durch das zu testende Gerät ausgeblasen, die zu testende Meßeinrichtung entnimmt eine Probe des Gasgemisches.

Die Mischkonzentrationen sind

- 20% Inertgas$^*$0.8(=80% des Mischbehälters)=16%
- 0.3% Diffusionsgas $^*$0.5(=50% des Mischbehälters)=0.15%

Das theoretisch zu erwartende Ergebnis errechnet sich aus der Formel

Diffusion = (53.6/10Sek)*log(0.3%Diffusionsgas $^*$ 16%Inertgas / 0.15%Diffusionsgas / 20%Inertgas) =1.09

Zur Durchführung eignet sich eine Pumpe mit 3 Ventileingängen, über die volumengetriggert 1. Umgebungsluft, 2. erstes Testgas und 3. zweites Testgas zugeführt werden.

**Patentansprüche**

1.   System zur Simulation eines CO Diffusionsmanövers bestehend aus einem manuell oder elektrisch befüllten Mischbehälter, gegebenenfalls mit Pumpenfunktion, mit steuerbaren Ventilen und einer Volumenmeßeinrichtung **dadurch gekennzeichnet,** dass der Mischbehälter volumengesteuert und/oder volumenabhängig mit verschiedenen Testgasen gefüllt wird, und dieses Gasgemisch der zu überprüfenden Meßeinrichtung zugeführt wird, welche dann funktions- und / oder bestimmungsgemäß verwendet wird.